# EUROPEAN PATENT SPECIFICATION

(11) **EP 2 976 078 B1**
(45) Date of publication and mention of the grant of the patent: **11.12.2019**
(21) Application number: 14770074.4
(22) Date of filing: 21.03.2014
(51) Int. Cl.: A61K 31/44, A61P 25/02

(54) **TREATMENT OF CHEMOTHERAPY-INDUCED PERIPHERAL NEUROPATHY**
BEHANDLUNG VON CHEMOTHERAPIE-INDUZIERTER PERIPHERER NEUROPATHIE
TRAITEMENT D'UNE NEUROPATHIE PÉRIPHÉRIQUE INDUITE PAR UNE CHIMIOTHÉRAPIE

(30) Priority: 21.03.2013 US 201313848262
(43) Date of publication of application: 27.01.2016
(73) Proprietor: Ghanbari, Hossein, Potomac, MD 20854 (US)
(72) Inventor: GHANBARI, Hossein, A., Potomac, MD 20854 (US); JIANG, Zhi-gang, Gaithersburg, MD 20878 (US)
(74) Representative: FRKelly
(86) International application number: PCT/US2014/031435
(87) International publication number: WO 2014/153505

(56) References cited:
- WO-A2-2006/076681
- US-A1- 2006 160 826
- US-A1- 2008 039 471
- US-A1- 2008 058 284
- US-A1- 2009 286 799
- US-A1- 2010 087 439
- US-A1- 2014 235 580
- US-A1- 2016 151 339
- CAROZZI ET AL.: 'The role of glutamate In diabetic and In chemotherapy Induced peripheral neuropathies and Its regulation by glutamate carboxypeptidase II.' CURR. MED. CHEM. vol. 19, no. 9, 2012, pages 1261 - 8, XP055279524 Retrieved from the Internet: <URL:http://www.ncbi.nim.nih.gov/pubmed/223 04715> [retrieved on 2014-10-10]

## Description

### BACKGROUND OF THE INVENTION

Chemotherapy-induced peripheral neuropathy (CIPN) is one of the most common, serious side effects that can lead to dose reductions or early discontinuation of chemotherapy, reducing the efficacy of cancer treatments. It can cause debilitating symptoms and also significantly impacts the patient's quality of life. An estimated 30 to 40 percent of cancer patients treated with chemotherapy experience CIPN.

The peripheral nervous system (PNS) consists of sensory neurons running from stimulus receptors that inform the central nervous system (CNS) of the stimuli, and motor neurons running from the spinal cord to the effectors that take action. In CIPN, an anticancer drug could impair both sensory and motor functions. The symptoms usually start in the hands and/or feet and creep up the arms and legs. Sometimes it feels like a tingling or numbness. Other times, it's more of a shooting and/or burning pain or sensitivity to temperature. It can include sharp, stabbing pain. CIPN can also lead to hearing loss, blurred vision and change in taste. CIPN can make it difficult to perform normal day-today tasks like buttoning a shirt, sorting coins in a purse, or walking. In addition, the motor neuron dysfunction manifest as cramps, difficulty with fine motor activities (e.g. writing or dialing a phone), gait disturbances, paralysis, spasms, tremors and weakness.

CIPN may result from the use of numerous chemotherapeutic agents, including, but limited to, Ixabepilone (Ixempra Kit), arsenic trioxide (Trisenox), cytarabine (Cytosar-U, Depocyt, generics), etoposide, hexamethylmelamine (altretamine [Hexalen]), Ifosfamide (Ifex, generics), methotrexate (Trexall, generics), procarbazine (Matulane) and vinblastine. The chemotherapeutic drugs that most commonly elicit CIPN include platinum compounds (cisplatin, carboplatin, oxaliplatin), vincristine, taxanes (docetaxel, paclitaxel), epothilones (ixabepilone), bortezomib (Velcade), thalidomide (Thalomid) and lenalidomide.

For treating the pain associated with CIPN, agents that appear promising include the antidepressants duloxetine and venlafaxine, which are both serotonin/norepinephrine-reuptake inhibitors. Another promising agent is a topical compound of the muscle-relaxant baclofen, the antidepressant amitriptyline, and the analgesic ketamine. Outside of clinical trials, CIPN symptoms are commonly managed in a manner similar to other types of nerve pain-that is, with a combination of physical therapy, complementary therapies such as massage and acupuncture, and medications that can include steroids, antidepressants, anti-epileptic drugs, and opioids for severe pain. But these therapies have not demonstrated true efficacy for CIPN, and virtually all of the drugs to treat peripheral neuropathy carry side effects of their own.

The actual causes of CIPN, on the cellular and tissue level, is still largely a matter of speculation. Oxidative stress may play a key role in CIPN. It was found that antioxidant machinery (e.g. plasma glutathione (GSH) and α- and γ-tocopherol concentrations) of cancer patients with chemotherapy decreased and the GSH redox state became more oxidized. In a rat model of painful oxaliplatin-induced neuropathy, oxidative stress was found to be an important component that mediates pain. In the plasma of oxaliplatin-treated rats, the increases of carbonylated protein and thiobarbituric acid reactive substances in the sciatic nerve and the spinal cord indicated the resultant protein oxidation and lipoperoxidation in these locations, respectively. Oxidative imbalance manifests itself as a mediator of inflammatory pain as well. Use of the anticancer drug cisplatin results in severe cell death of sensory neurons derived from dorsal root ganglia following increase in oxidative stress. Oxidative stress is also found to impair the autonomic nervous system and manifests itself in symptoms such as hearing loss. The results from antioxidants also support a key role of oxidative stress in mediating CIPN. The antineuropathic effect of antioxidant silibinin or α-tocopherol shows as about 50% oxaliplatin-induced behavioral alterations. Administration of anticancer drug bortezomib or oxaliplatin, which elicits TRPA1-dependent hypersensitivity, produced a rapid, transient increase in plasma of carboxy-methyllysine, a by-product of oxidative stress. Short-term systemic treatment with either HC-030031 or α-lipoic acid (an antioxidant) could completely prevent hypersensitivity if administered before the cytotoxic drug. The findings highlight a key role for early activation/sensitization of TRPA1 by oxidative stress by-products in producing CIPN. For preventing the onset of CIPN, further clinical testing of many antioxidative stress agents, such as glutathione, acetyl-L-carnitine and alpha-lipoic acid has been suggested.

Another mechanism underlying CIPN is excitotoxicity where increased release of glutamate forces N-methyl D-aspartate (NMDA) receptors to remain open, allowing increased calcium flux into neurons, resulting in overexcitation and eventually neuronal rupture. The end result of this process is pain without a painful stimulus, also known as neuropathic pain. N-Acetyl-aspartyl-glutamate (NAAG) is an abundant neuropeptide widely distributed in the central and peripheral nervous system which is physiologically hydrolyzed by the enzyme glutamate carboxypeptidase into N-Acetyl-aspartyl (NAA) and glutamate. Glutamate carboxypeptidase inhibition could reduce the severity of chemotherapy-induced peripheral neurotoxicity in rat.

As there are no proven treatments, there is a need for methods to properly treat chemotherapy-induced peripheral neuropathy. The present invention provides just such a method.

### SUMMARY OF THE INVENTION

The present invention is directed to compositions for use in a method of treating chemotherapy-induced peripheral neuropathy.

One embodiment of the present invention is directed to compositions for use in a method of treating chemotherapy-induced peripheral neuropathy, the use comprising administering to a patient in need at least one thiosemicarbazone compound that is 3-aminopyridine-2-carboxaldehyde thiosemicarbazone

Another embodiment of the present invention is directed to compositions for use in a method of treating chemotherapy-induced peripheral neuropathy, the use comprising administering to a patient in need a composition comprising 3-aminopyridine-2-carboxaldehyde thiosemicarbazone, wherein the step of administering is intravenous, intraperitoneal, subcutaneous, intramuscular, topical, transdermal or oral.

The present invention further encompasses compositions for use in methods of treating chemotherapy-induced peripheral neuropathy, the use comprising administering a composition comprising a compound of Formula I 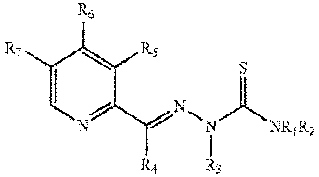
wherein R₁, R₂, R₃, and R₄ are independently selected from the group consisting of hydrogen, C1-8alkyl, C2-8alkenyl, C2-8alkynyl, C3-8cycloalkyl, C1-8haloalkyl, C6-10 aryl, amino-C1-8 alkyl, hydroxy-C1-8 alkyl, C1-8 alkoxy-C1-8 alkyl, and C1- 8alkanoyl;or NR₁R₂ taken in combination form a 3 to 7 member ring which may comprise 0, 1, or 2 additional ring heteroatoms selected from N, 0, and S;
R₆ is hydrogen, hydroxy, amino, or C1-8alkyl; and
R₅ and R₇ are independently selected from the group consisting of hydrogen, halide, hydroxy, thiol, amino, hydroxyamino, mono-C1-8alkylamino, di(C1-8 alkyl)amino, C1-8 alkoxy, C1-8alkyl, C1-8 alkenyl, and C2-8alkynyl.

The present invention further encompasses compositions for use in methods of treating chemotherapy-induced peripheral neuropathy, the use comprising administering a composition comprising a compound of Formula II:

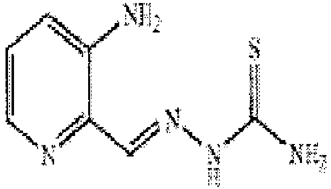

### DETAILED DESCRIPTION OF THE INVENTION

For simplicity and illustrative purposes, the principles of the present invention are described by referring to various exemplary embodiments thereof. Before explaining the disclosed embodiments of the present invention in detail, it is to be understood that the invention is not limited in its application to the details of any particular arrangement shown, since the invention is capable of other embodiments. The terminology used herein is for the purpose of description and not of limitation. Further, although certain methods are described with reference to certain steps that are presented herein in certain order, in many instances, these steps may be performed in any order as would be appreciated by one skilled in the art, and the methods are not limited to the particular arrangement of steps disclosed herein.

The present invention is directed to a composition for use in a method for the treatment of chemotherapy induced peripheral neuropathy, the use comprising the step of administering to a patient a composition comprising a thiosemicarbazone compound as recited in Claim 1. The means for synthesis of thiosemicarbazone compounds useful in the methods of the invention are well known in the art. Such synthetic schemes are described in U.S. Pat. Nos. 5,281,715; 5,767,134; 4,447,427; 5,869,676 and 5,721,259.

The chemical structures of PAN-811's analogues are shown in U.S. Pat. No 7,456,179, and patent applications of 20090275587, 20060194810 and 20060160826.

The pharmaceutical compositions for use required by the present invention typically comprise a compound useful in the invention and a pharmaceutically acceptable carrier. As used herein "pharmaceutically acceptable carrier" includes any and all solvents, dispersion media, coatings, antibacterial and antifungal agents, isotonic and absorption delaying agents, and the like that are physiologically compatible. The type of carrier can be selected based upon the intended route of administration. In various embodiments, the carrier is suitable for intravenous, intraperitoneal, subcutaneous, intramuscular, topical, transdermal or oral administration. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, monostearate salts and gelatin. Moreover, the compounds can be administered in a time release formulation, for example in a composition which includes a slow release polymer. The active compounds can be prepared with carriers that will protect the compound against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid and polylactic, polyglycolic copolymers (PLG). Many methods for the preparation of such formulations are generally known to those skilled in the art.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Depending on the route of administration, the compound may be coated in a material to protect it from the action of enzymes, acids and other natural conditions which may inactivate the agent. For example, the compound can be administered to a subject in an appropriate carrier or diluent co-administered with enzyme inhibitors or in an appropriate carrier such as liposomes. Pharmaceutically acceptable diluents include saline and aqueous buffer solutions. Enzyme inhibitors include pancreatic trypsin inhibitor, diisopropylfluoro-phosphate (DEP) and trasylol. Liposomes include water-in-oil-in-water emulsions as well as conventional liposomes. Dispersions can also be prepared in glycerol, liquid polyethylene glycols, and mixtures thereof and in oils. Under ordinary conditions of storage and use, these preparations may contain a preservative to prevent the growth of microorganisms.

The active agent in the composition (i.e. one or more thiosemicarbazones) preferably is formulated in the composition in a therapeutically effective amount. A "therapeutically effective amount" refers to an amount effective, at dosages and for periods of time necessary, to achieve the desired therapeutic result to thereby influence the therapeutic course of a particular disease state. A therapeutically effective amount of an active agent may vary according to factors such as the disease state, age, sex, and weight of the individual, and the ability of the agent to elicit a desired response in the individual. Dosage regimens may be adjusted to provide the optimum therapeutic response. A therapeutically effective amount is also one in which any toxic or detrimental effects of the agent are outweighed by the therapeutically beneficial effects. In another embodiment, the active agent is formulated in the composition in a prophylactically effective amount. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

The amount of active compound in the composition may vary according to factors such as the disease state, age, sex, and weight of the individual. Dosage regimens may be adjusted to provide the optimum therapeutic response. For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

A compound of the invention can be formulated into a pharmaceutical composition wherein the compound is the only active agent therein. Alternatively, the pharmaceutical composition can contain additional active agents. For example, two or more compounds of the invention may be used in combination.

3-aminopyridine-2-carboxaldehyde thiosemicarbazone (hereinafter "PAN-811"), with a molecular weight of 195.24 Da, has demonstrated potent neuroprotective activities in several neurodegenerative models. PAN-811 was originally developed for cancer therapy due to its ability to inhibit ribonucleotide reductase, a key enzyme required for DNA synthesis. Our previous studies demonstrated that PAN-811 at concentration of 0.45 µM fully blocked ischemic neurodegeneration and at 1.2µM completely halted hypoxia-induced neuronal cell death. PAN-811 was administered intracerebroventricularly (*i.c.v.*) at a dose of 50 µg per rat at 1 h after arterial occlusion. Staining of consecutive brain sections and computer-assisted quantitative analysis demonstrated that PAN-811 reduced the infarct volume by 59% in PAN-811 treated rats. We also investigated the effect of a single intravenous (i.v.) bolus injection of PAN-811. Two-hour middle cerebral artery occlusion (MCAo) with cerebral blood flow reduction of 75% or greater resulted in infarct formation, brain edema and a significant number of premature deaths. PAN-811 treatment reduced infarct volume in a dose dependent manner with a maximal protection of 50% at a dose of 2mg/kg. PAN-811 treatment (2mg/kg) also resulted in a 70% reduction in brain edema volume. Accordingly, the mortality in PAN-811 treated groups was collectively reduced by 44% (Jiang et al., 2008). Mechanistically PAN-811 prevents glutamate-induced excitatory cytotoxicity, veratridine-induced sodium channel opening that is related to Ca²⁺ influx and staurosporine-induced apoptosis. Nearly complete neuroprotection against glutamate insult is observed in cultured neuronal cells if the cells were pretreated with 10µM PAN-811 for 24 h. In culture, ischemic condition results in a 19-fold increase in intracellular free calcium. PAN-811 at a dose of 5µM reduced this elevated level by 72%. In a cell-free system by taking EDTA as a positive control, PAN-811 chelates free calcium as efficiently as EDTA. In addition, PAN-811 effectively suppresses oxidative stress in many ways. PAN-811 at a concentration as low as 1µM suppressed in vitro hydrogen peroxide-induced LDH release by 78% (with P<0.01, compared to untreated/H₂0₂- insulted group) and at a concentration of 10 µM achieved maximal protection (by 90% comparing with untreated and H₂0₂-insulted group) with an EC₅₀ of about 0.55µ.M. PAN-811 also inhibited oxidative stress-induced cell death of human Alzheimer's disease-derived and age-matched olfactory neuroepithelial cells via suppression of intracellular reactive oxygen species. Importantly, PAN-811 manifested as a free radical scavenger in a cell free system where PAN-811 reduced 500µM of a stable free radical diphenylpicrylhydrazyl by 70%. Taken together, PAN-811 has manifested as a potent neuroprotectant with dual drug targets - oxidative stress and free calcium.

Based on the key roles of excitoneurotoxicity and oxidative stress in chemotherapy-induced peripheral neuropathy and also the potent free calcium chelating and antioxidative effects of PAN-811, we have discovered that PAN-811 is a therapeutic agent for chemotherapy-induced peripheral neuropathy. PAN-811 should inhibit chemotherapy-induced peripheral neuropathy that is not only caused with antimetabolites (cytarabine, gludarabine, fluorouracil, mercaptopurine, methotrexate, thioguanine, gemcitabine, hydroxyurea), mitotic inhibitors (vincristine, vinblastine, vinorelbine), topoisomerase inhibitors (topotecan, irenotecan), paclitaxel, docetaxel and asparaginase, but also with alkylating agents (busulfan, carmustine, lomustine, chlorambucil, cyclophosphamide, cisplatin, carboplatin, oxaliplatin, ifosamide, mechlorethamine, melphalan, thiotepa, dacarbazine, procarbazine), antitumor antibiotics (bleomycin, dactinomycin, daunorubicin, doxorubicin, idarubicin, mitomycin, mitoxantrone, plicamycin), topoisomerase II inhibitor (etoposide, teniposide), and radiation therapy. In addition, PAN-811 should inhibit chemotherapy-induced peripheral neuropathy caused by other anticancer drug, such as ixabepilone, arsenic trioxide, etoposide, hexamethylmelamine, ifosfamide, methotrexate, procarbazine, epothilones, bortezomib, thalidomide and lenalidomide.

While the invention has been described with reference to certain exemplary embodiments thereof, those skilled in the art may make various modifications to the described embodiments of the invention, in as fas as these are covered by the scope of the attached claims.

## Claims

1. A composition for use in a method for the treatment of chemotherapy induced peripheral neuropathy, wherein the composition comprises 3-aminopyridine-2-carboxaldehyde thiosemicarbazone (PAN-811) or at least one thiosemicarbazone compound (Formula I): 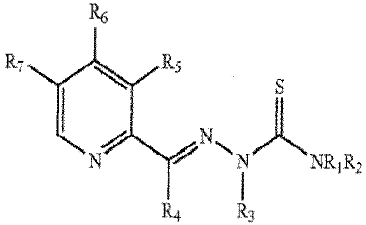
wherein R₁, R₂, R₃, and R₄ are independently selected from the group consisting of hydrogen, C1-8alkyl, C2-8alkenyl, C2-8alkynyl, C3-8cycloalkyl, C1-8haloalkyl, C6-10aryl, amino-C1-8 alkyl, hydroxy-C1-8alkyl, C1-8alkoxy-C1-8alkyl, and C1-8alkanoyl;or NR₁R₂ taken in combination form a 3 to 7 member ring which may comprise 0, 1, or 2 additional ring heteroatoms selected from N, 0, and S;
R₆ is hydrogen, hydroxy, amino, or C1-8alkyl; and
R₅ and R₇ are independently selected from the group consisting of hydrogen, halide, hydroxy, thiol, amino, hydroxyamino, mono-C1-8alkylamino, di(C1-8 alkyl)amino, C1-8 alkoxy, C1-8alkyl, C1-8 alkenyl, and C2-8 alkynyl.

2. The composition for use of claim 1, wherein the at least one thiosemicarbazone compound comprises the compound of Formula II:

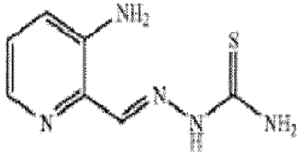

3. The composition for use of claim 1, wherein the step of administering is intravenous, intraperitoneal, subcutaneous, intramuscular, topical, transdermal or oral.

4. The composition for use of claim 1, wherein the composition is an injectable and/or infusible solution.

5. The composition for use of claim 1, wherein the composition is formulated as a micro emulsion.

6. The composition for use of claim 1, wherein the composition is formulated as a liposome.

## Patentansprüche

1. Zusammensetzung zur Verwendung in einem Verfahren zur Behandlung einer durch Chemotherapie induzierten peripheren Neuropathie, wobei die Zusammensetzung 3-Aminopyridin-2-carboxaldehyd-thiosemicarbazon (PAN-811) oder mindestens eine Thiosemicarbazon-Verbindung (Formel I) umfasst: 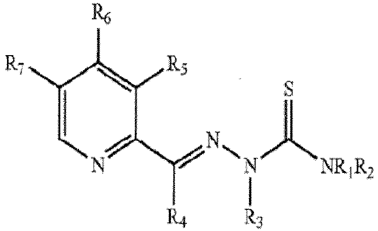
wobei R₁, R₂, R₃ und R₄ unabhängig ausgewählt sind aus der Gruppe bestehend aus Wasserstoff, C1-8-Alkyl, C2-8-Alkenyl, C2-8-Alkynyl, C3-8-Cycloalkyl, C1-8-Haloalkyl, C6-10-Aryl, C1-8-Aminoalkyl, C1-8-Hydroxyalkyl, C1-8-Alkoxy-C1-8-Alkyl und C1-8-Alkanoyl; oder NR₁R₂ in Kombination einen 3- bis 7-gliedrigen Ring bilden, der 0, 1 oder 2 zusätzliche Ring-Heteroatome umfassen kann, die ausgewählt sind aus N, 0 und S;
R₆ Wasserstoff, Hydroxy, Amino oder C1-8-Alkyl ist; und
R₅ und R₇ unabhängig ausgewählt sind aus der Gruppe bestehend aus Wasserstoff, Halogenid, Hydroxy, Thiol, Amino, Hydroxyamino, Mono-C1-8-Alkylamino, Di(C1-8-Alkyl)amino, C1-8-Alkoxy, C1-8-Alkyl, C1-8-Alkenyl und C2-8-Alkynyl.

2. Zusammensetzung zur Verwendung nach Anspruch 1, wobei die mindestens eine Thiosemicarbazon-Verbindung die Verbindung von Formel II umfasst:

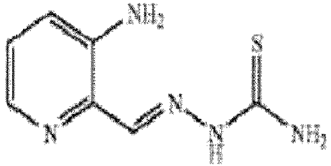

3. Zusammensetzung zur Verwendung nach Anspruch 1, wobei der Schritt des Verabreichens intravenös, intraperitoneal, subkutan, intramuskulär, topisch, transdermal oder oral ist.

4. Zusammensetzung zur Verwendung nach Anspruch 1, wobei die Zusammensetzung eine injizierbare und/oder infundierbare Lösung ist.

5. Zusammensetzung zur Verwendung nach Anspruch 1, wobei die Zusammensetzung als eine Mikroemulsion formuliert ist.

6. Zusammensetzung zur Verwendung nach Anspruch 1, wobei die Zusammensetzung als ein Liposom formuliert ist.

## Revendications

1. Composition pour une utilisation dans une méthode de traitement d'une neuropathie périphérique induite par une chimiothérapie, la composition comprenant de la 3-aminopyridine-2-carboxaldéhyde thiosemicarbazone (PAN-811) ou au moins un composé de thiosemicarbazone (Formule I) : 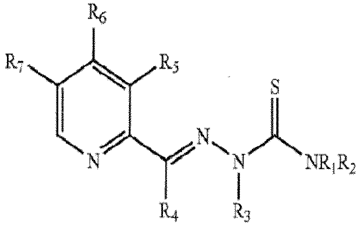
dans laquelle R₁, R₂, R₃ et R₄ sont indépendamment choisis dans le groupe consistant en hydrogène, alkyle en C1-8, alcényle en C2-8, alcynyle en C2-8, cycloalkyle en C3-8, haloalkyle en C1-8, aryle en C6-10, amino-alkyle en C1-8, hydroxy-alkyle en C1-8, alcoxy en C1-8-alkyle en C1-8 et alcanoyle en C1-8 ; ou NR₁R₂ pris en combinaison forment un cycle à 3 à 7 chaînons qui peut comprendre 0, 1 ou 2 hétéroatomes de cycle supplémentaires choisis parmi N, O et S ;
R₆ est hydrogène, hydroxy, amino ou alkyle en C1-8 ; et
R₅ et R₇ sont indépendemment choisis dans le groupe consistant en hydrogène, halogénure, hydroxy, thiol, amino, hydroxyamino, mono-alkyl en C1-8-amino, di(alkyl en C1-8)amino, alcoxy en C1-8, alkyle en C1-8, alcényle en C1-8 et alcynyle en C2-8.

2. Composition pour l'utilisation selon la revendication 1, dans laquelle ledit au moins un composé de thiosemicarbazone comprend le composé de Formule II :

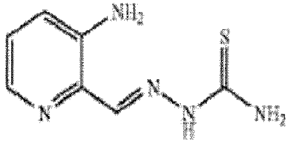

3. Composition pour l'utilisation selon la revendication 1, dans laquelle l'étape d'administration est intraveineuse, intrapéritonéale, sous-cutanée, intramusculaire, topique, transdermique ou orale.

4. Composition pour l'utilisation selon la revendication 1, dans laquelle la composition est une solution injectable et/ou perfusable.

5. Composition pour l'utilisation selon la revendication 1, dans laquelle la composition est formulée sous la forme d'une micro émulsion.

6. Composition pour l'utilisation selon la revendication 1, dans laquelle la composition est formulée sous la forme de liposomes.
